# EUROPEAN PATENT SPECIFICATION

(11) **EP 4 062 929 B1**
(45) Date of publication and mention of the grant of the patent: **09.07.2025**
(21) Application number: 20889462.6
(22) Date of filing: 18.11.2020
(51) Int. Cl.: A61K 39/00, A61P 25/00, A61P 25/28, A61P 37/04, C07K 14/47, A61K 47/60, G01N 33/50, G01N 33/53, G01N 33/531, G01N 33/543, G01N 33/547

(54) **CROSSLINKED PRODUCT OF AMYLOID-BETA PROTEIN (A-BETA) ABLE TO BE A SUBSTITUTE FOR AMYLOSPHEROID (ASPD), AND ANALYSIS OF ASPD**
VERNETZTES PRODUKT VON AMYLOID-BETA PROTEIN (A-BETA). DAS EIN ERSATZ FÜR AMYLOSPHOID (ASPD) SEIN KANN, UND ANALYSE VON ASPD
PRODUIT RÉTICULÉ DE LA PROTÉINE AMYLOÏDE-BETA (A-BETA) POUVANT ÊTRE SUBSTITUÉ PAR L'AMYLOSPHÉROÏDE (ASPD), ET ANALYSE DE L'ASPD

(30) Priority: 19.11.2019 JP 2019209136
(43) Date of publication of application: 28.09.2022
(73) Proprietor: Foundation for Biomedical Research and Innovation at Kobe, Kobe-shi, Hyogo 650-0047 (JP)
(72) Inventor: HOSHI Minako, Kyoto 606-8501 (JP); ARAI Yoshie, Kyoto 606-8501 (JP); MACHIDA Kiyotaka, Osaka-shi, Osaka 540-0021 (JP)
(74) Representative: Dehns
(86) International application number: PCT/JP2020/042938
(87) International publication number: WO 2021/100744

(56) References cited:
- EP-A1- 2 224 000
- WO-A1-2009/057664
- WO-A1-2017/179646
- WO-A2-99/41279
- JP-A- 2008 291 025
- JP-A- 2008 531 635
- JP-A- 2019 518 196
- US-A1- 2009 246 191
- US-A1- 2011 104 821
- HITOMI KOMURA ET AL: "Alzheimer A[beta] Assemblies Accumulate in Excitatory Neurons upon Proteasome Inhibition and Kill Nearby NAK[alpha]3 Neurons by Secretion", ISCIENCE, vol. 13, March 2019 (2019-03-01), pages 452 - 477, XP055624336
- MATSUZAKI K ET AL: "Design, synthesis, and biophysical properties of a helical A[beta]1-42 analog: Inhibition of fibrillogenesis and cytotoxicity", BIOCHEMICAL AND BIOPHYSICAL RESEARCH COMMUNICATIONS, vol. 371, no. 4, 2008, pages 777 - 780, XP022688462
- MURAKAMI K: "Conformation-specific antibodies to target amyloid [beta] oligomers and their application to immunotherapy for Alzheimer's disease", BIOSCIENCE, BIOTECHNOLOGY, AND BIOCHEMISTRY, vol. 78, no. 8, 2014, pages 1293 - 1305, XP055927833

## Description

### Technical Field

The present disclosure relates to a cross-linked product of amyloid-β protein (AB), a cross-linked Aβ that can be a substitute for amylospheroids (ASPD), and analysis of ASPD.

### Background Art

Amylospheroids (ASPD; amylospheroids) are each a spherical AB assembly that is formed of about 30 numbers of amyloid-β proteins (AB) aggregated together and has a diameter of about 10 to 15 nm. ASPD are structures considered to play an important role in neuronal cell death in Alzheimer's disease.

ASPD were isolated as in vitro-synthesized Aβ aggregates (i.e., synthetic ASPD) that are highly neurotoxic (Non-Patent Document 1). Antibodies specific for the synthetic ASPD were produced (Patent Documents 1 and 2), and using these antibodies, ASPD formed in vivo (that is, native ASPD) were actually isolated from the brains of human patients with Alzheimer's disease (Non-Patent Document 1).

Both native ASPD and synthetic ASPD induce cell death selectively in mature neuronal cells. The target of ASPD in this neuronal cell death was found to be "α3 subunit of Na⁺, K⁺-ATPase pump (hereafter referred to as "NAKα3")", which is a synaptic protein playing a crucial role in neuronal survival and function, and it was revealed that the ion pump function of NAKα3 is impaired owing to binding of ASPD thereto, thereby causing excessive excitation of neuronal cells leading to the death of those neuronal cells (Patent Document 3 and Non-Patent Document 2).

The loss of neurons is most highly correlated with clinical symptoms of Alzheimer's disease. It was revealed that, in Alzheimer's disease patients with loss of neurons, the amount of native ASPD in the cerebral cortex increases in correlation with the severity of the Alzheimer's disease, whereas only a trace amount of native ASPD is seen in the cerebellum of Alzheimer's disease patients with little loss of neurons (Non-Patent Document 3). Accordingly, it is considered that ASPD play an important role in the irreversible stage in the development of Alzheimer's disease. Furthermore, native ASPD were also detected in the brains of Lewy body dementia patients (Non-Patent Document 3), and this suggests that ASPD also play an important role in the development of Lewy body dementia.

Synthetic ASPD considered to be equivalent to native ASPD can be produced by slowly stirring a liquid containing AB (Non-Patent Document 1 and Patent Document 4).

Also, cell secreted-type ASPD-like structures, which are considered to be equivalent to native ASPD, can be obtained by being secreted from, for example, CHO cells that express any kinds of APP proteins as described below, and can be obtained from culture supernatant of the CHO cells (Patent Document 5). Non-Patent Document 4 discloses that Alzheimer Aβ assemblies accumulate in excitatory neurons upon proteasome inhibition and kill nearby NAKα3 neurons by secretion.

### Citation List

### Patent Documents

Patent Document 1: WO 2006/016644
Patent Document 2: WO 2009/057664
Patent Document 3: WO 2013/099806
Patent Document 4: WO 2013/094614
Patent Document 5: WO 2017/179646

### Non-Patent Documents

Non-Patent Document 1: Hoshi et al., Spherical aggregates of β-amyloid (amylospheroid) show high neurotoxicity and activate tau protein kinase I/glycogen synthase kinase-3β, PNAS May 27, 2003 vol. 100 no. 11 6370-6375
Non-Patent Document 2: Ohnishi et al., Na, K-ATPase α3 is a death target of Alzheimer patient amyloid-β assembly, PNAS August 11, 2015 vol. 112 no. 32 E4465-E4474
Non-Patent Document 3: Noguchi et al., Isolation and characterization of patient-derived, toxic, high mass amyloid beta-protein (Abeta) assembly from Alzheimer disease brains, J Biol Chem. 2009 Nov 20; 284 (47): 32895-905
Non-Patent Document 4 : Komura et al., Alzheimer Aβ assemblies accumulate in excitatory neurons upon proteasome inhibition and kill nearby NAKα3 neurons by secretion, iScience, 2019 vol. 13, 452-477

### Disclosure of Invention

### Problem to be Solved by the Invention

As described above, amylospheroids (ASPD) play an important role in Alzheimer's disease and Lewy body dementia. There is a growing need for analyzing ASPD in biospecimens from subjects (patients) for prevention, diagnostics, treatment, and the like of diseases in which ASPD are involved.

In addition, if a therapeutic agent that targets ASPD is developed, ASPD analysis would be necessary for companion diagnostics.

Analysis (including measurement) of a substance generally requires a reference material (standard). The reference material is used for, for example, calibration or preparation of a calibration curve. Native ASPD, synthetic ASPD, and cell secreted-type ASPD-like structures can be used as a reference material for ASPD analysis.

On the other hand, in the case of a reference material to be included in a diagnostic kit, it is desirable that the reference material can be produced stably, has excellent storage stability, and is produced at a low production cost. As a reference material to be included in a kit for ASPD analysis, native ASPD, synthetic ASPD, and cell secreted-type ASPD-like structures have problems in terms of ease of production, storage stability, production cost, and the like.

Thus, viewed from one aspect, the present disclosure provides a substance that can be a substitute for ASPD or a substance that can be a reference material for ASPD in ASPD analysis.

Viewed from another aspect, the present disclosure provides a method capable of analyzing ASPD in a biospecimen such as, for example, blood or cerebrospinal fluid.

### Solution to Problem

Viewed from one aspect, the present disclosure relates to a substance that competes with ASPD against an ASPD-specific antibody,
the substance being a substance in which amyloid-β protein (AB) is cross-linked with a cross-linking agent,
wherein the cross-linking agent is a cross-linking agent that has a spacer arm length of between 4 Å and 50 Å inclusive or a cross-linking agent that has, as a spacer arm, not less than 1 and not more than 13 numbers of an oxyethylene group (-CH₂CH₂O-) and/or an oxypropylene group (-CH₂CH₂CH₂O-).

Viewed from another aspect, the present disclosure relates to a cross-linked product of Aβ, wherein the AB is cross-linked using a cross-linking agent that has a spacer arm length of between 4 Å and 50 Å inclusive or a cross-linking agent that has, as a spacer arm, not less than 1 and not more than 13 groups that are an oxyethylene group and/or an oxypropylene group.

The present invention relates to a method for analyzing ASPD, including the steps of:
(1) bringing a biological sample that may contain ASPD into contact with an antibody (first antibody) that is capable of binding to ASPD and is bound to an insoluble carrier, thereby causing, when the biological sample contains ASPD, the ASPD to bind to the first antibody; and
(2) reacting an anti-ASPD monoclonal antibody (second antibody) with the ASPD that has bound to the first antibody in the step (1),

wherein the first antibody is a humanized anti-ASPD monoclonal antibody huASD2 or a monoclonal antibody BAN50a, and
the second antibody is an anti-ASPD monoclonal antibody mASD3 or a labeled antibody that is mASD3 with a label.

### Advantageous Effects of Invention

Viewed from one aspect, the present disclosure can provide a substance that can be a substitute for ASPD or a substance that can be a reference material for ASPD in ASPD analysis.

Viewed from another aspect, the present disclosure can provide a method that can analyze ASPD in a biospecimen such as, for example, blood or cerebrospinal fluid.

### Brief Description of Drawings

[FIG. 1] FIG. 1 shows an example of the results of SDS-PAGE for cross-linked Aβ1, cross-linked Aβ2, and cross-linked Aβ3.
[FIG. 2] FIG. 2 is a schematic view illustrating one embodiment of ASPD CLEIA [anti-ASPD polyclonal antibody/mASD3].
[FIG. 3] FIG. 3 shows an example of a graph comparing the reactivities of ASPD with that of the cross-linked Aβ1, cross-linked Aβ2, and cross-linked Aβ3, respectively using ASPD CLEIA.
[FIG. 4] FIG. 4 shows examples of graphs comparing the quantification of the cross-linked Aβ1, ASPD, and an Aβ1-16 dimer using a commercially available Aβ oligomer ELISA with those using an ASPD CLEIA.
[FIG. 5] FIG. 5 shows an example of the results of examining the immunogenicity of the cross-linked Aβ1, and the results demonstrate that the serum of a rabbit immunized with the cross-linked Aβ1 reacts with synthetic ASPD immobilized on the plate.
[FIG. 6] FIG. 6 shows an example of a graph obtained by plotting measured values obtained for diluted human CSF samples containing ASPD (dilution ratios: 1, 1/2, 1/4, and 1/8) in measurement by ASPD CLEIA.
[FIG. 7] FIG. 7 shows an example of a graph obtained by plotting measured values obtained for diluted human plasma samples containing ASPD (dilution ratios: 1, 1/2, 1/4, 1/8, and 1/16) in measurement by ASPD CLEIA.
[FIG. 8] FIG. 8 is a schematic view illustrating one embodiment of ASPD CLEIA [huASD2/mASD3].
[FIG. 9] FIG. 9 shows an example of a graph showing the results of measurement on four independent ASPD-containing samples by ASPD CLEIA [huASD2/mASD3].
[FIG. 10] FIG. 10 is a schematic view illustrating one embodiment of ASPD CLEIA [huASD2/biotinylated mASD3].
[FIG. 11] FIG. 11 shows an example of a graph showing the results of measurement on ASPD-containing samples by ASPD CLEIA [huASD2/biotinylated mASD3].
[FIG. 12] FIG. 12 shows examples of graphs showing the results of detecting ASPD and MAP16 using an ASPD CLEIA [huASD2/biotinylated mASD3] system and a [BAN50/biotinylated BAN50] system, respectively.
[FIG. 13] FIG. 13 shows examples of the result of comparing the results obtained for a human plasma sample (dilution ratio: 1/4) and a buffer sample when using various combinations of [first antibody/second antibody].
[FIG. 14] FIG. 14 shows an example of the results of performing ASPD CLEIA [huASD2/biotinylated mASD3] for plasma samples from 10 patients diagnosed as having Alzheimer's disease (AD) and plasma samples from 10 healthy subjects.

### Description of the Invention

Viewed from one aspect, the present disclosure is based on the finding that a cross-linked product of Aβ obtained through cross-linking with the use of a predetermined cross-linking agent exhibits reactivity similar to that of ASPD against ASPD-specific antibodies.

Viewed from another aspect, the present disclosure is based on the finding that ASPD in a biospecimen can be efficiently analyzed using a predetermined combination of antibodies.

In the present disclosure, the term "ASPD" per se may encompass native ASPD, synthetic ASPD, and cell secreted-type ASPD-like structures.

Examples of the ASPD-specific antibody include, in one or more embodiments, polyclonal anti-ASPD antibodies and monoclonal anti-ASPD antibodies disclosed in WO 2006/016644 and WO 2009/057664, and in one or more other embodiments, rabbit polyclonal anti-ASPD antibodies (rpASD1, rpASD2, and rpASD3), mouse monoclonal anti-ASPD antibodies (mASD1, mASD2, and mASD3), hamster monoclonal anti-ASPD antibodies (haASD1, haASD2, haASD3, haASD4, and haASD5), and a humanized monoclonal anti-ASPD antibody (huASD2).

### Amyloid-β proteins

AB is a protein that consists of a sequence corresponding to part of an amyloid precursor protein (APP) and has the sequence of a peptide cleaved from APP with β-secretase and γ-secretase in vivo.

In the present disclosure, the term "Aβ" per se may refer to Aβ38 (Aβ1-38), Aβ39 (AB1-39), Aβ40 (AB1-40), Aβ41 (AB1-41), Aβ42 (AB1-42), or Aβ43 (AB1-43), or all of them or only one or some of them.

In the present disclosure, the term "Aβ" per se may encompass human Aβ and nonhuman mammal AB.

In one or more embodiments, examples of human APP include splicing variants such as hAPP770, hAPP751, and hAPP695. Specific examples thereof include, but not limited to, hAPP770 (NCBI Accession No. NP_000475 [VERSION NP_000475.1 GI: 4502167]).

The N-terminal amino acid of human Aβ (the first amino acid in AB) corresponds to amino acid D (aspartic acid) at position 672 in hAPP770.

In the present disclosure, the term "Aβ" per se may encompass wild-type Aβ and mutant AB.

In one or more embodiments, examples of the mutant AB include those having the sequence of a peptide cleaved from mutant APP. The mutant APP may be APP with a mutation linked to familial Alzheimer's disease, and examples of the mutation include the Swedish mutation, the Leuven mutation, the Icelandic mutation, the London mutation, the Iranian mutation, the Austrian mutation, the German mutation, the French mutation, the Florida mutation, the Iberian mutation, the Australian mutation, the Belgian mutation, the Flemish mutation, the Icelandic mutation, the British mutation, the Tottori mutation, the Italian mutation, the Arctic mutation, the Osaka mutation, the Iowa mutation, and the Dutch mutation. Other examples of the mutation include mutations to a modified amino acid, an unnatural amino acid, a D-amino acid, or the like. In one or more embodiments, the modified amino acid may be an amino acid modified on its amino group, carboxyl group, thiol group, or hydroxy group or modified by glycation, PEGylation, or the like.

### Cross-linked product of Aβ (Cross-linked AB)

Viewed from one aspect, the present disclosure relates to a cross-linked Aβ obtained through cross-linking of Aβ using a predetermined cross-linking agent. AB is as described above.

The cross-linking agent used in the cross-linked AB of the present disclosure is, in one or more embodiments, a cross-linking agent that has a spacer arm length of between 4 Å and 50 Å inclusive. The spacer arm length refers to the molecular span (the distance between bonding molecules) of the cross-linking agent. Since catalogs and instructions for use of commercially available cross-linking agents indicate the spacer arm length of the cross-linking agents, those skilled in the art can select and obtain the cross-linking agent having a desired spacer arm length.

In one or more embodiments, the spacer arm length is 4 Å or more, preferably 7 Å or more, preferably 10 Å or more, more preferably 13 Å or more, more preferably 14 Å or more, more preferably 15 Å or more, still more preferably 16 Å or more, still more preferably 17 Å or more, still more preferably 18 Å or more, still more preferably 19 Å or more, still more preferably 20 Å or more, and still more preferably 21 Å or more, from the viewpoint of allowing the reactivity of the cross-linked AB with ASPD-specific antibodies to be closer to that of ASPD.

In one or more embodiments, the spacer arm length is 50 Å or less, preferably 45 Å or less, more preferably 40 Å or less, still more preferably 36 Å or less, yet more preferably 33 Å or less, even more preferably 30 Å or less, even more preferably 27 Å or less, and even more preferably 24 Å or less, from the same viewpoint.

In one or more embodiments, the cross-linking agent used in the cross-linked AB according to the present disclosure is a cross-linking agent that has, as a spacer arm, not less than 1 and not more than 13 groups that are an oxyethylene group(s) (-CH₂CH₂O-, EO group) and/or an oxypropylene group(s) (-CH₂CH₂CH₂O-, PO group). The spacer arm refers to the skeleton or group present between reactive ends of the cross-linking agent.

In one or more embodiments, the total number of EO groups and PO groups in the spacer arm is 1 or more, preferably 2 or more, more preferably 3 or more, and still more preferably 4 or more, from the viewpoint of allowing the reactivity of the cross-linked AB with ASPD-specific antibodies to be closer to that of ASPD.

In one or more embodiments, the total number of EO groups and PO groups in the spacer arm is 13 or less, preferably 10 or less, more preferably 9 or less, still more preferably 8 or less, yet more preferably 7 or less, and even more preferably 6 or less, from the same viewpoint.

In one or more embodiments, the cross-linking target of the cross-linking agent used in the cross-linked AB according to the present disclosure may be, for example, an amino group, a sulfhydryl group, or a carboxyl group, or may be nonselective.

In one or more embodiments, the reactive ends of the cross-linking agent used in the cross-linked AB according to the present disclosure are preferably reactive ends that cross-link between amino groups from the viewpoint of allowing the reactivity of the cross-linked AB with ASPD-specific antibodies to be closer to that of ASPD, and such reactive ends may be NHS esters or imidoesters.

The cross-linking agent used in the cross-linked AB according to the present disclosure may or may not be cleavable. From the viewpoint of stability, it is preferable that the cross-linking agent is not cleavable.

In one or more embodiments, the cross-linking agent used in the cross-linked AB according to the present disclosure is preferably ethylene glycol bis(succinimidyl succinate) (EGS, spacer arm length: 16.1 Å), sulfo-EGS (spacer arm length: 16.1 Å), bis(succinimidyl) penta(ethylene glycol) (BS(PEG)₅, spacer arm length: 21.7 Å), or bis(succinimidyl) nona(ethylene glycol) (BS(PEG)₉, spacer arm length: 35.8 Å), from the viewpoint of allowing the reactivity of the cross-linked AB with ASPD-specific antibodies to be closer to that of ASPD. Of these, BS(PEG)₅ and BS(PEG)₉ are more preferable, and BS(PEG)₅ is still more preferable.

Aβ used in the cross-linked AB according to the present disclosure may be Aβ38, Aβ39, Aβ40, Aβ41, or Aβ42 from the viewpoint of allowing the reactivity of the cross-linked AB with ASPD-specific antibodies to be closer to that of ASPD. Of these, Aβ40 or Aβ42 is preferable, and Aβ42 is more preferable.

In one or more embodiments, the molecular weight of the cross-linked AB according to the present disclosure is more than 100 kDa or more than 114 kDa, with the molecular weight being a value obtained through analysis by 10% to 20% SDS-PAGE.

In one or more embodiments, the cross-linked AB according to the present disclosure may have cytotoxicity (i.e., the properties of selectively inducing cell death of functionally mature neuronal cells) equivalent to that of ASPD, or may be less toxic than ASPD. Alternatively, the cross-linked AB may be free of cytotoxicity or may be more toxic than ASPD. In one or more embodiments, the cross-linked AB according to the present disclosure may be less toxic than ASPD or is free of cytotoxicity.

In one or more embodiments, the reactivity of the cross-linked Aβ according to the present disclosure with ASPD-specific antibodies is closer to that of ASPD than to those of AB aggregates other than ASPD, and more preferably is equivalent to that of ASPD.

In one or more embodiments, the cross-linked Aβ according to the present disclosure is a substance that competes with ASPD against ASPD-specific antibodies.

In one or more embodiments, the cross-linked Aβ according to the present disclosure can be a substitute for ASPD from the viewpoint of reactivity with ASPD-specific antibodies.

In one or more embodiments, the cross-linked AB according to the present disclosure can be a reference material for ASPD analysis or a reference material to be included in a kit for ASPD analysis.

In the present disclosure, analysis of ASPD may include, in one or more embodiments, detection, measurement, and quantification of ASPD, and screening utilizing them.

In one or more embodiments, ASPD analysis in which the cross-linked Aβ according to the present disclosure is used as a reference material may be analysis utilizing an immunoassay that uses an ASPD-specific antibody(ies). Such analysis may be specifically an enzyme immunoassay that uses an ASPD-specific antibody(ies) or an enzyme immunoassay that is based on chemiluminescence and use an ASPD-specific antibody(ies), and more specifically an enzyme-linked immunosorbent assay (ELISA) or a chemiluminescent enzyme immunoassay (CLEIA), each of which uses an ASPD-specific antibody(ies).

In one or more embodiments, the cross-linked Aβ according to the present disclosure can be used for preparation of a calibration curve in ASPD analysis. In one or more other embodiments, the cross-linked AB according to the present disclosure can be used as a positive control in ASPD analysis.

In one or more embodiments, the cross-linked AB according to the present disclosure can be a reference material for ASPD analysis in diagnostics of a disease involving accumulation of ASPD. In one or more embodiments, examples of the disease involving accumulation of ASPD include Alzheimer's disease and Lewy body dementia.

In one or more embodiments, the cross-linked Aβ according to the present disclosure can be a reference material for ASPD analysis in companion diagnostics for a therapeutic agent that targets ASPD, or a reference material to be included in a kit for this ASPD analysis. In one or more embodiments, the cross-linked AB according to the present disclosure can be a component of a companion diagnostic agent for a therapeutic agent that targets ASPD.

Thus, viewed from another aspect, the present disclosure relates to an ASPD analysis kit that includes the cross-linked AB according to the present disclosure as a reference material. In one or more embodiments, the ASPD analysis kit according to the present aspect includes a reagent(s) necessary for an immunoassay that uses an ASPD-specific antibody(ies). In one or more embodiments, examples of the reagent include ASPD-specific antibodies.

In one or more embodiments, the ASPD analysis kit according to the present aspect is an ASPD analysis kit for diagnostics of a disease involving accumulation of ASPD, an ASPD analysis kit for companion diagnostics for a therapeutic agent that targets ASPD, or a companion diagnostic agent for a therapeutic agent that targets ASPD.

In one or more embodiments, examples of the therapeutic agent that targets ASPD include pharmaceutical compositions for prevention, amelioration, and/or treatment of a disease involving accumulation of ASPD.

In one or more embodiments, the cross-linked AB according to the present disclosure is immunogenic and can induce production of an antibody against the cross-linked AB according to the present disclosure.

In one or more embodiments, the cross-linked Aβ according to the present disclosure is immunogenic and can induce production of antibodies against ASPD.

Accordingly, in one or more embodiments, the cross-linked AB according to the present disclosure can be a substitute for ASPD from the viewpoint of capability of inducing antibodies against ASPD.

Using ASPD as an active vaccine for a disease involving accumulation of ASPD has already been disclosed (e.g., WO 2017/179646).

Since the cross-linked AB according to the present disclosure can be a substitute for ASPD, the cross-linked AB according to the present disclosure can also be used as an active vaccine and also can be used in production of a vaccine.

Thus, viewed from one aspect, the present disclosure relates to a pharmaceutical composition containing the cross-linked Aβ according to the present disclosure as an active ingredient. Examples of one or more embodiments of the pharmaceutical composition according to the present disclosure include vaccines containing the cross-linked AB according to the present disclosure. The pharmaceutical composition and vaccine according to the present disclosure may contain a pharmaceutically acceptable excipient and/or adjuvant.

In one or more embodiments, the pharmaceutical composition and vaccine according to the present disclosure can be used for prevention, amelioration, and/or treatment of a disease involving accumulation of ASPD. In one or more embodiments, the pharmaceutical composition and vaccine according to the present disclosure can be used for prevention, amelioration, and/or treatment of Alzheimer's disease and/or Lewy body dementia.

In one or more embodiments, the pharmaceutical composition and vaccine according to the present disclosure can be administered to a subject who is at risk of developing, is suspected of having, or has a disease involving accumulation of ASPD. In one or more embodiments, the pharmaceutical composition according to the present disclosure can be administered to a subject who is at risk of developing, is suspected of having, or has Alzheimer's disease and/or Lewy body dementia. The subject may be a mammal, a human, or a non-human mammal.

One or more embodiments in which the pharmaceutical composition and vaccine according to the present disclosure are used to immunize a subject against ASPD, a disease involving accumulation of ASPD, or Alzheimer's disease and/or Lewy body dementia will be described below. In one or more embodiments, the method for administering the pharmaceutical composition and vaccine according to the present disclosure may be intramuscular, intraperitoneal, intradermal, or subcutaneous injection, or transmucosal administration through the oral tract, gastrointestinal tract, respiratory tract, or genitourinary tract. The dose of the cross-linked AB in the pharmaceutical composition and vaccine according to the present disclosure may be such an amount that a protective immune response is induced without causing severe adverse effects in a subject to which the pharmaceutical composition and vaccine is administered. In one or more embodiments, the dose of the pharmaceutical composition and vaccine according to the present disclosure is estimated to be an amount that contains a cell secreted-type ASPD-like structure in the range from 0.01 µg to 10 mg, 0.1 to 1000 µg, 1 to 100 µg, 5 to 50 µg, or 5 to 25 µg. Following administration of the initial dose, one or several booster doses may be administered at sufficient intervals.

Thus, viewed from one aspect, the present disclosure relates to a method for preventing, ameliorating, and/or treating a disease involving an accumulation of ASPD (e.g., Alzheimer's disease and/or Lewy body dementia), including administering to a subject the cross-linked AB according to the present disclosure or the pharmaceutical composition or vaccine according to the present disclosure.

Viewed from another aspect, the present disclosure relates to a method for immunizing a subject against ASPD themselves, including administering to the subject the cross-linked AB according to the present disclosure or the pharmaceutical composition or vaccine according to the present disclosure.

Viewed from still another aspect, the present disclosure relates to a method for immunizing a subject against a disease involving an accumulation of ASPD, including administering to the subject the cross-linked AB according to the present disclosure or the pharmaceutical composition or vaccine according to the present disclosure.

Furthermore, viewed from one aspect, the present disclosure relates to a method for producing the pharmaceutical composition or vaccine according to the present disclosure, including combining the cross-linked AB according to the present disclosure with an excipient and/or adjuvant.

Viewed from another aspect, the present disclosure relates to a method for producing a pharmaceutical composition containing the cross-linked AB according to the present disclosure as an active ingredient or for producing a vaccine containing the cross-linked AB according to the present disclosure, including the step of producing the cross-linked AB according to the present disclosure by a production method to be described below.

### Method for producing cross-linked Aβ

In one or more embodiments, the cross-linked Aβ according to the present disclosure can be obtained by adding a cross-linking agent to an aqueous AB solution in which Aβ is dissolved in an aqueous solvent to cause a cross-linking reaction.

AB may be prepared through peptide synthesis or may be produced using a recombination technique.

In one or more embodiments, the amount of the cross-linking agent to be added is not less than 10 times, not less than 20 times, or not less than 30 times the amount of Aβ on a molar basis. In one or more embodiments, the amount of the cross-linking agent to be added is not more than 200 times, not more than 100 times, or not more than 75 times the amount of Aβ on a molar basis.

A cross-linked product obtained after the cross-linking reaction may be further subjected to ultrafiltration. The cross-linked AB according to the present disclosure can be obtained in retention liquid or in recovery liquid containing filter residues.

In one or more embodiments, the molecular weight cut-off value (MWCO) of an ultrafiltration filter may be 30 kDa, 50 kDa, or 100 kDa.

In one or more embodiments, the cross-linked Aβ according to the present disclosure can be produced by the method described in examples.

The following listed cross- linked AB [A1] to [A10] may be used in methods of the invention, as described hereinafter.

[A1] A cross-linked Aβ wherein AB is cross-linked using a cross-linking agent that has a spacer arm length of between 4 Å and 50 Å inclusive.

[A2] A cross-linked Aβ wherein AB is cross-linked using a cross-linking agent that has, as a spacer arm, not less than 1 and not more than 13 groups that are an EO group and a PO group.

[A3] The cross-linked AB according to [A1] or [A2], wherein Aβ is Aβ38, Aβ39, Aβ40, Aβ41, Aβ42, or Aβ43.

[A4] The cross-linked AB according to any one of [A1] to [A3], wherein the cross-linking agent has reactive ends that cross-link between amino groups.

[A5] The cross-linked AB according to any one of [A1] to [A4], having a molecular weight of more than 100 kDa with the molecular weight being a value obtained through analysis by 10% to 20% SDS-PAGE.

[A6] The cross-linked AB according to any one of [A1] to [A5], which is a substance that competes with ASPD against an ASPD-specific antibody.

[A7] The cross-linked AB according to any one of [A1] to [A6], which is a reference material in ASPD analysis.

[A8] The cross-linked AB according to any one of [A1] to [A7], which is a reference material for ASPD analysis in diagnostics of a disease involving accumulation of ASPD.

[A9] The cross-linked AB according to any one of [A1] to [A8], which is a reference material for ASPD analysis in companion diagnostics for a therapeutic agent that targets ASPD.

[A10] The cross-linked AB according to any one of [A1] to [A9], which is a component of a companion diagnostic agent for a therapeutic agent that targets ASPD.

### ASPD analysis method

Viewed from one aspect, the present disclosure relates to a method for analyzing ASPD, including the following steps (1) and (2) (hereinafter also referred to as "ASPD analysis method according to the present disclosure"):
(1) bringing a biological sample that may contain ASPD into contact with an antibody (first antibody) that is capable of binding to ASPD and is bound to an insoluble carrier, thereby causing, when the biological sample contains ASPD, the ASPD to bind to the first antibody; and
(2) reacting an anti-ASPD monoclonal antibody (second antibody) with the ASPD that has bound to the first antibody in the step (1).

In this method, the first antibody is a humanized anti-ASPD monoclonal antibody huASD2 or a monoclonal antibody BAN50a, and
the second antibody is a mouse monoclonal anti-ASPD antibody mASD3 or a labeled antibody that is mASD3 with a label.

The ASPD analysis method according to the present disclosure is a method that utilizes the principle of an immunoassay, and in one or more embodiments, ASPD analysis method is an enzyme immunoassay such as ELISA or a chemiluminescent enzyme immunoassay such as CLEIA.

In one or more embodiments, the first antibody to be bound to the insoluble carrier is preferably huASD2 or BAN50a from the viewpoint of improving the detection sensitivity when blood, plasma components, cerebrospinal fluid, or a diluted solution thereof is used as a sample.
huASD2, which is disclosed in WO 2009/057664, is a humanized monoclonal anti-ASPD antibody having heavy chain variable regions whose amino acid sequence is represented by SEQ ID NO: 1 in the Sequence Listing and light chain variable regions whose amino acid sequence is represented by SEQ ID NO: 2 in the Sequence Listing.
SEQ ID NO: 1: 
EVQLVESGGGLVKPGGSLRLSCAASGFTFSDYFMSWVRQAPGKGLEWVGGIEIK
SYFYATYYFGSVKGRFTISRDDSKNTLYLQMNSLKTEDTAVYYCTTNREVGGLDN
 WGQGTLVTVSS
SEQ ID NO: 2: 
QSVLTQPSSLSASPGASASLTCTLRSGISVGGKNIYWYQQKPGSPPQYLLKYSSYS
NKQLGPGVPSRFSGSKDASANAGILLISGLQSEDEADYYCSIHESNAYVFGGGTK
LTVLGR
 huASD2 can be obtained by, for example, co-transfecting ASD2RHApG1D200 (NITE Patent Microorganisms Depositary, Accession number: FERM BP-11040) and ASD2RLApLN100 (NITE Patent Microorganisms Depositary, Accession number: FERM BP -11041) into known animal cells to cause the cells to express them.

In one or more embodiments, it is possible to use, instead of huASD2 in the present disclosure, an anti-ASPD antibody having heavy chain variable regions whose amino acid sequence is represented by SEQ ID NO: 3 in the Sequence Listing and light chain variable regions whose amino acid sequence is represented by SEQ ID NO: 4 in the Sequence Listing.
SEQ ID NO: 3: 
EVQLVESGGGLVKPGGSLRLSCAASGFTFSDYFMSWVRQAPGKGLEWVXGIEIK
SYFYATYYFGSVKGRFTISRDDSKNTXYLQMNSLKTEDTAVYYCTXNREVGGLD
NWGQGTLVTVSS
SEQ ID NO: 4: 
QXVLTQPXSLSASPGASASLTCTLRSGISVGGKNIYWYQQKPGSPPQXXLXYSSYS
NKQLGPGVPSRFSGSKDXSANAXILLISGLQSEDEADYYCSIHESNAYVFGGGTK
LTVLG
(In SEQ ID NO: 3, X at position 49 is G or A, X at position 81 is L or V, and X at position 100 is T or R. In SEQ ID NO: 4, X at position 2 is S or A, X at position 8 is S or A, X at position 48 is Y or F, X at position 49 is L or F, X at position 51 is K, F, or R, X at position 74 is A or T, and X at position 79 is G or A.)

In the present disclosure, BAN50a is a known monoclonal antibody that specifically recognizes the N-terminal region ofAB and is disclosed in WO 1994/17197. Commercially available products can be used as BAN50a, or BAN50a can be produced by a known method using Hybridoma BAN50 (Accession number: FERM BP4163) deposited in NITE Patent Microorganisms Depositary.

In one or more embodiments, the second antibody may be a labeled antibody that is mASD3 with a label from the viewpoint of improving the detection sensitivity. In one or more embodiments, labeling may be achieved through biotinylation.

In one or more embodiments, examples of the insoluble carrier used in the ASPD analysis method according to the present disclosure include solid phase materials such as beads and multi-well plates.

In one or more embodiments, examples of the biological sample that may contain ASPD include blood, plasma, and cerebrospinal fluid, as well as dilutions thereof.

In one or more embodiments, the ASPD analysis method according to the present disclosure may include a step (0) of preparing the biological sample prior to the step (1). In one or more embodiments, this sample preparation step (0) includes diluting a biospecimen such as blood, plasma, or cerebrospinal fluid with a suitable solvent or a suitable buffer. In one or more embodiments, the solvent or buffer used in this sample preparation step (0) may contain a surfactant and/or any other drug to the extent that the analysis is not hindered.

In one or more embodiments, the ASPD analysis method according to the present disclosure may further includes the following step (3):
(3) reacting, with the second antibody that has bound to the ASPD in the step (2), an anti-mouse antibody having a reporter linked thereto, or reacting, with a label of the second antibody that has bound to the ASPD in the step (2), a binding partner of the label, having a reporter linked thereto.

In one or more embodiments, the reporter may be a substance that utilizes chemiluminescence or bioluminescence, a fluorescent protein, or the like. From the viewpoint of improving the detection sensitivity, the reporter is preferably a substance that utilizes chemiluminescence or bioluminescence.

In one or more embodiments, the substance that utilizes chemiluminescence or bioluminescence may be an enzyme such as a bioluminescent enzyme or a variant thereof.

In one or more embodiments, examples of the enzyme include HRP (horseradish peroxidase) and alkaline phosphatase.

In one or more embodiments, the binding partner may be streptavidin or the like when the label is biotin.

In one or more embodiments, measurement of luminescence can be performed using a luminometer or a multi-plate reader.

In one or more embodiments, the ASPD analysis method according to the present disclosure may use the cross-linked AB according to the present disclosure as a reference material.

As disclosed hereinbefore, the invention relates to a method for analyzing ASPD, including the following steps (1) and (2):
(1) bringing a biological sample that may contain ASPD into contact with an antibody (first antibody) that is capable of binding to ASPD and is bound to an insoluble carrier, thereby causing, when the biological sample contains ASPD, the ASPD to bind to the first antibody; and
(2) reacting an anti-ASPD monoclonal antibody (second antibody) with the ASPD that has bound to the first antibody in the step (1).

In this method, the first antibody is a humanized anti-ASPD monoclonal antibody huASD2 or a monoclonal antibody BAN50a, and
the second antibody is a mouse monoclonal anti-ASPD antibody mASD3 or a labeled antibody that is mASD3 with a label.

In a preferred aspect, this analysis method is a method utilizing the principle of an immunoassay.

In a further preferred aspect of this analysis method, the method utilizes the principle of an immunoassay is ELISA or CLEIA.

In a preferred aspect of any of the above analysis methods, the biological sample is blood, plasma components, cerebral spinal fluid, or a diluted solution thereof.

In a further preferred aspect of any of the above analysis methods, the method further includes a step (0) of preparing the biological sample prior to the step (1).

In a preferred aspect of the above analysis method, the sample preparation step (0) includes diluting a biospecimen selected from the group consisting of blood, plasma, and cerebrospinal fluid with a solvent or a buffer.

In a further preferred aspect of any of the above analysis methods , the method further includes the following step (3):
(3) reacting, with the second antibody that has bound to the ASPD in the step (2), an anti-mouse antibody having a reporter linked thereto, or reacting, with a label of the second antibody that has bound to the ASPD in the step (2), a binding partner of the label, having a reporter linked thereto.

In a preferred aspect of any of the above analysis methods, a substance according to [1] described below is used as a reference material for ASPD or the method includes using the cross-linked AB according to any one of [A1] to [A10] as described hereinbefore as a reference material.

Substances and cross-linked products that may be used in methods of the invention as described hereinbefore and hereinafter are set out below:.
[1] A substance that competes with ASPD against an ASPD-specific antibody,
   the substance being a substance in which Aβ is cross-linked with a cross-linking agent,
   wherein the cross-linking agent is a cross-linking agent that has a spacer arm length of between 4 Å and 50 Å inclusive or a cross-linking agent that has, as a spacer arm, not less than 1 and not more than 13 groups that are an EO group and/or a PO group.
[2] A cross-linked product of Aβ, wherein the Aβ is cross-linked using a cross-linking agent that has a spacer arm length of between 4 Å and 50 Å inclusive or a cross-linking agent that has, as a spacer arm, not less than 1 and not more than 13 groups that are an EO group and/or a PO group.

The method of the invention for analyzing ASPD, includes the steps of:
(1) bringing a biological sample that may contain ASPD into contact with an antibody (first antibody) that is capable of binding to ASPD and is bound to an insoluble carrier, thereby causing, when the biological sample contains ASPD, the ASPD to bind to the first antibody; and
(2) reacting an anti-ASPD monoclonal antibody (second antibody) with the ASPD that has bound to the first antibody in the step (1),

wherein the first antibody is a humanized anti-ASPD monoclonal antibody huASD2 or a monoclonal antibody BAN50a, and
the second antibody is a mouse monoclonal anti-ASPD antibody mASD3 or a labeled antibody that is mASD3 with a label.

In a preferred aspect the above analysis method further comprises the step of:
(3) reacting, with the second antibody that has bound to the ASPD in the step (2), an anti-mouse antibody having a reporter linked thereto, or reacting, with a label of the second antibody that has bound to the ASPD in the step (2), a binding partner of the label, having a reporter linked thereto.

In a yet further preferred aspect, the analysis method described above uses the substance according to [1] described hereinbefore or the cross-linked product according to [2] as described herein before as a reference material for ASPD.

In the following, one or more embodiments of the present invention will be further described with reference to examples.

### Examples

In the following examples, the concentration of a cross-linked AB and the concentration of ASPD are AB monomer-equivalent concentrations, unless otherwise stated.

### 1. Preparation of cross-linked Aβs

Cross-linked Aβ1, cross-linked Aβ2, and cross-linked Aβ3 shown below were prepared.
Cross-linked Aβ1: Aβ42 with BS(PEG)₅ as a cross-linking agent
Cross-linked Aβ2: Aβ42 with BS(PEG)₉ as a cross-linking agent
Cross-linked Aβ3: Aβ42 with DFDNB as a cross-linking agent

The cross-linking agents used were as follows.
BS(PEG)₅: Bis(succinimidyl) penta(ethylene glycol)
(spacer arm length: 21.7 Å)
BS(PEG)₉: Bis(succinimidyl) nona(ethylene glycol)
(spacer arm length: 35.8 Å)
DFDNB: 1,5-difluoro-2,4-dinitrobenzene
(spacer arm length: 3.0 Å)

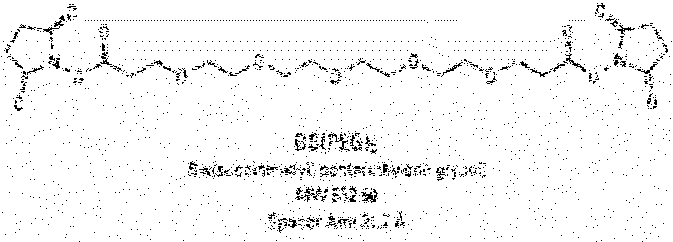

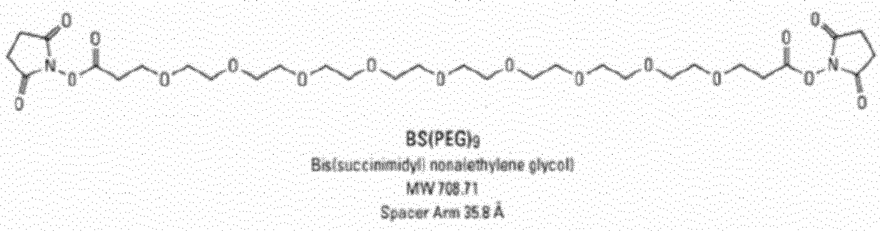

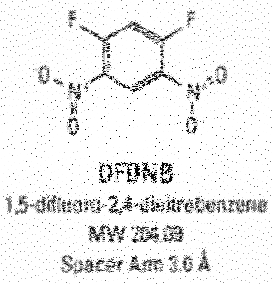

### Protocol for cross-linked Aβs

### Dissolve 0.56 mg of Aβ42 in 120 µL of DMSO (1 mM)

↓ Add 10 µL of the resultant solution to 90 µL of PBS (-), and then allow the resultant mixture to stand still at 25°C for 15 minutes (100 µM of Aβ, which corresponds to 0.046667 mg)
↓ Add a cross-linking reagent BS(PEG)₅, BS(PEG)₉, or DFDNB in an amount of 50 times by mole, and then allow the resultant mixture to stand still at 25°C for 30 minutes
↓ Add Tris-HCl (pH 8.0) thereto (final concentration: 50 mM), and then allow the resultant mixture to stand still at 25°C for 30 minutes
↓ Filter the mixture through an ultrafiltration filter with a MWCO of 50K to obtain a high molecular weight fraction
   Wash the high molecular weight fraction three times with 300 µL of PBS (-)
   Collect membrane residues with 50 µL of PBS (-)
↓ Measure the concentration by a BCA method

### 2. SDS-PAGE of cross-linked Aβs

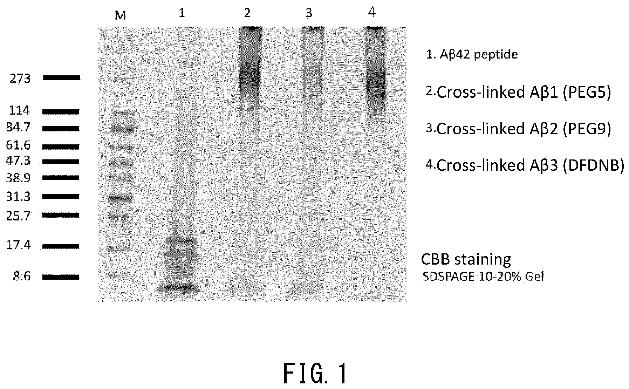

The cross-linked Aβ1, cross-linked Aβ2, and cross-linked Aβ3 obtained were subjected to SDS-PAGE (10% to 20% Gel) to check whether they were cross-linked. FIG. 1 shows an example of the results obtained.

As can be seen from FIG. 1, the cross-linked AB 1 to 3 were each found to be polymerized and to have a molecular weight of 100 kDa or more.

### 3. Reactivities of cross-linked Aβs in ASPD CLEIA

Reactivities of the obtained cross-linked Aβ1, cross-linked Aβ2, and cross-linked Aβ3 in ASPD CLEIA were examined.

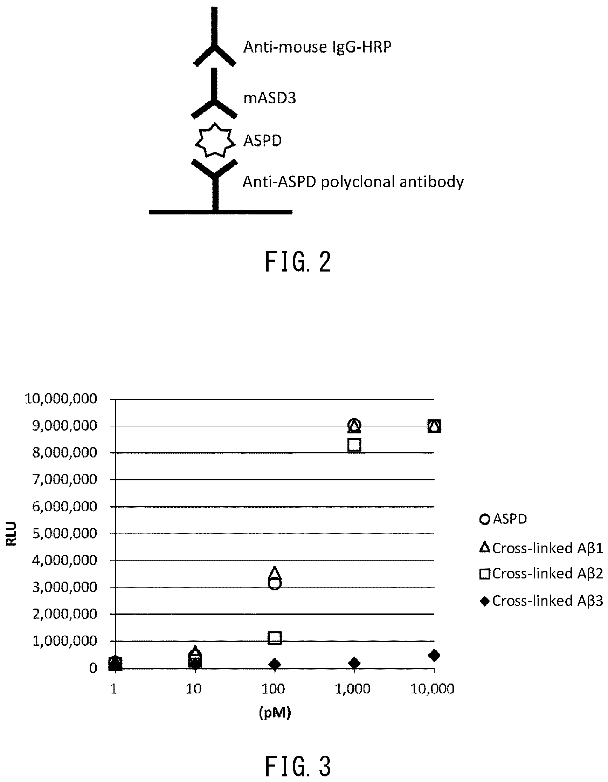

ASPD CLEIA used was the system shown in FIG. 2, in which an anti-ASPD polyclonal antibody (first antibody) immobilized on a solid phase, a mouse anti-ASPD monoclonal antibody mASD3 (second antibody) as a detection-side antibody, and an anti-mouse IgG-HRP were used. mASD3 is a monoclonal antibody described in WO 2006/016644 and WO 2009/057664, and a hybridoma for mASD3 has been deposited in NITE Patent Microorganisms Depositary (Accession number: FERM BP-10394). HRP stands for "horseradish peroxidase".

The reactivity in ASPD CLEIA [anti-ASPD polyclonal antibody/mASD3] when each of the cross-linked Aβ1, cross-linked Aβ2, and cross-linked Aβ3 was used as an analyte was compared with the reactivity in ASPD CLEIA when ASPD were used as an analyte, and FIG. 3 shows an example of the results obtained.

As can be seen from FIG. 3, the reactivities of the cross-linked Aβ1 and cross-linked Aβ2 were closer to the reactivity of ASPD than the reactivity of the cross-linked Aβ3 was. In particular, the reactivity of the cross-linked Aβ1 was equivalent to the reactivity of ASPD.

### Comparison of reactivity of cross-linked AB (cont.)

Subsequently, the reactivity of a cross-linked AB was compared using a commercially available Aβ oligomer measurement kit that uses a monoclonal antibody 82E 1, which specifically recognizes the N-terminus of Aβ.

The cross-linked Aβ1, ASPD, and an Aβ1-16 dimer were used as analytes.

Furthermore, reactivities of the cross-linked Aβ1, ASPD, and the Aβ1-16 dimer in ASPD CLEIA [anti-ASPD polyclonal antibody/mASD3] were also compared.

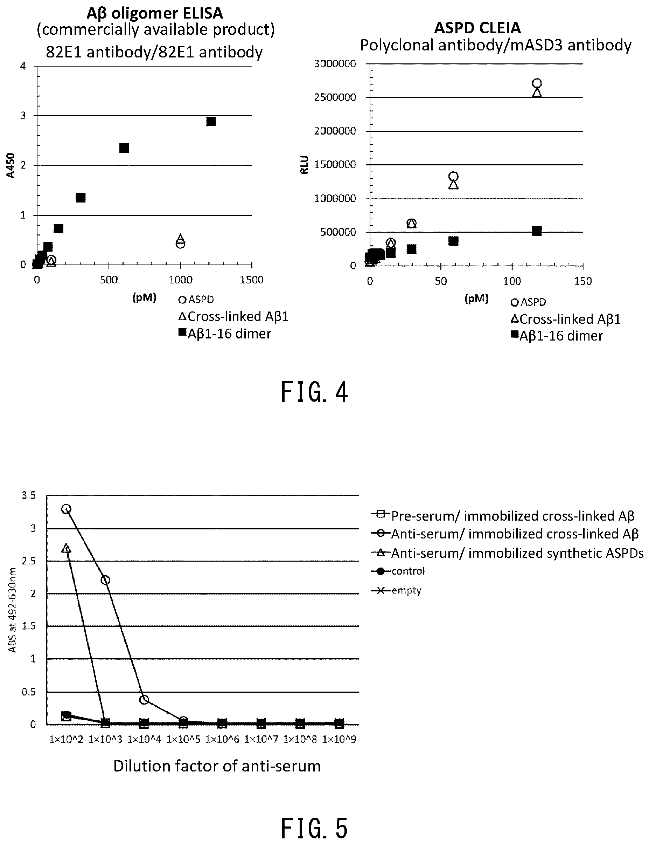

FIG. 4 shows an example of the results obtained.

As can be seen from FIG. 4, when the commercially available Aβ oligomer measurement kit was used, the cross-linked Aβ1 also exhibited the reactivity equivalent to that of ASPD, similarly to the case of ASPD CLEIA.

In the case where the commercially available Aβ oligomer measurement kit using 82E1 was used, the reactivities of the cross-linked Aβ1 and ASPD were much lower than the reactivity of the Aβ1-16 dimer.

In contrast, in ASPD CLEIA, the reactivity of the Aβ1-16 dimer was much lower than the reactivities of the cross-linked Aβ1 and ASPD.

Moreover, it was also found that ASPD CLEIA exhibits high detection sensitivity for the cross-linked Aβ1 and ASPD.

### 4. Immunogenicity of cross-linked Aβs

Rabbits (New Zealand White) were immunized six times with the cross-linked Aβ1 (100 µg/single immunization) together with Freund's adjuvant (FCA, first time) and incomplete Freund's adjuvant (FIA, five times), followed by exsanguination. Then, the reactivities of antibodies contained in serum with the cross-linked AB 1 and a synthetic ASPD were evaluated. FIG. 5 shows an example of the results obtained.

In FIG. 5, "pre-serum" indicates the reactivity of pre-immune serum with a plate having the cross-linked Aβ1 immobilized thereon, "immobilized cross-linked Aβ" indicates the reactivity of the obtained serum with a plate having the cross-linked Aβ1 immobilized thereon, "immobilized synthetic ASPD" indicates the reactivity of the obtained serum with a plate having synthetic ASPD immobilized thereon, "control" indicates the reactivity of the obtained serum with a plate having a F12 protein immobilized thereon, and "Empty" indicates the reactivity of the obtained serum with an empty plate.

The results shown in FIG. 5 demonstrate that the cross-linked AB 1 is immunogenic and can induce antibodies reactive with ASPD.

### 5. Measurement of ASPD in cerebrospinal fluid (CSF)

ASPD in human CSF specimens were measured using an ASPD CLEIA system [anti-ASPD polyclonal antibody/mASD3]. The flow of the measurement was specifically as follows.
(1) Add known concentrations of ASPD to CSF, and dilute the resultant solutions with a specimen diluent (1- to 16-fold dilution).
(2) First reaction: add each of the resultant diluted solutions to a multi-well plate having a rabbit anti-ASPD polyclonal antibody immobilized thereon at 100 µL/well, and allow the plate to stand still at 25°C for 60 minutes.
   Washing × 3
(3) Second reaction: add mASD3 (1 µg/ml) at 100 µL/well, and allow the plate to stand still at 25°C for 60 minutes.
   Washing × 3
(4) Third reaction: add anti-mouse IgG-HRP (1/20000) at 100 µL/well, and allow the plate to stand still at 25°C for 60 minutes.
   Washing × 3
(5) Measurement: add a chemiluminescent substrate (product name: SuperSignal pico, Thermo Fisher) for HRP at 100 µL/well, and measure the luminescence using a plate reader (product name: infinite M200pro, TECAN).

As a standard for measurement, the cross-linked Aβ1 or a cell secreted-type ASPD-like structure (WO 2017/179646) was used.

As a specimen diluent, 3% BSAin PBS (-) with 0.05% antiseptic (product name: ProClin, Sigma-Aldrich) was used.

PBS (-) with 0.05% Tween 20 was used as a washing solution.

Table 1 below shows one example of the results obtained when known concentrations of ASPD (high concentration H: 108 pM, medium concentration M: 54 pM, low concentration L: 27.5 pM, and none N: 0 pM) were added to human CSF (Age 84/Caucasian/M), the resultant solutions were diluted 4-fold, and the thus-obtained diluted solutions were subjected to quantification.

**[Table 1]**

| Recovery rate of ASPD added to human CSF (Dilution factor: × 4) | | | | |
|---|---|---|---|---|
| | Concentration of Added ASPD (pM) | Theoretical Value (pM) | Measured Value (pM) | Recovery Rate % |
| H | 108 | 108 | 110.8 | 103 |
| M | 54 | 54 | 51.4 | 95 |
| L | 27.5 | 27.5 | 29.5 | 107 |
| N | 0 | 0 | 0 | 0 |

As can be seen from Table 1, a recovery rate of approximately 100% was achieved. Similar results were obtained when human CSF (Age 66/Caucasian/M) and human CSF (Age 69/Caucasian/M) were used.

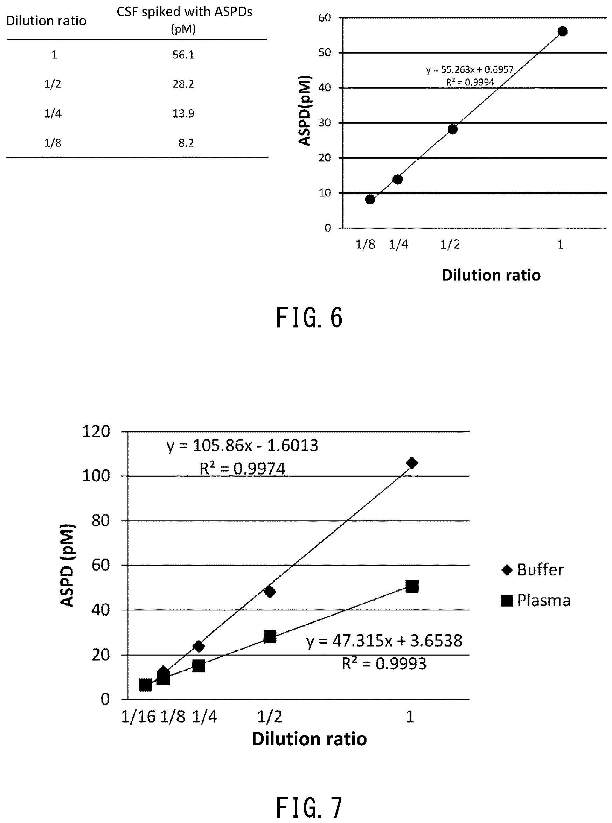

Next, FIG. 6 shows the results obtained when human CSF was spiked with ASPD to prepare a sample, the sample was then diluted 2-fold, 4-fold, and 8-fold with a specimen diluent, and the samples with the dilution ratios of 1, 1/2, 1/4, and 1/8 were subjected to measurement.

As can be seen from FIG. 6, dilution linearity was observed as a result of plotting the results obtained for the diluted samples.

### 6. Measurement of ASPD in human plasma

ASPD in human plasma specimens were measured using ASPD CLEIA [anti-ASPD polyclonal antibody/mASD3]. The flow of the measurement was specifically as follows.
(1) Add known concentrations of ASPD to human plasma, and dilute the resultant solutions with a specimen diluent (1- to 16-fold dilution).
(2) First reaction: add the resultant diluted solutions to a multi-well plate having a rabbit anti-ASPD polyclonal antibody immobilized thereon at 100 µL/well, and shake the plate at 25°C for 60 minutes.
   Washing × 3
(3) Second reaction: add mASD3 (1 µg/ml) at 100 µL/well, and shake the plate at 25°C for 60 minutes.
   Washing × 3
(4) Third reaction: add anti-mouse IgG-HRP (1/20000) at 100 µL/well, and shake the plate at 25°C for 60 minutes.
   Washing × 3
(5) Measurement: add a chemiluminescent substrate (product name: SuperSignal pico, Thermo Fisher) for HRP at 100 µL/well, and measure the luminescence using a plate reader (product name: infinite M200pro, TECAN).

As a standard for measurement, the cross-linked Aβ1 or a cell secreted-type ASPD-like structure (WO 2017/179646) was used.

As a specimen diluent, 3% BSAin PBS (-) with 0.05% antiseptic (product name: ProClin, Sigma-Aldrich) was used.

PBS (-) with 0.05% Tween20 was used as a washing solution.

Table 2 below shows one example of the results obtained when known concentrations of ASPD were added to human plasma (Age 29/Caucasian/F), the resultant solutions were diluted 4-fold with 3% BSA PBS (-), and the obtained diluted solutions were subjected to quantification.

**[Table 2]**

| Recovery rate of ASPD added to human plasma (dilution factor: × 4) | | | | |
|---|---|---|---|---|
| | Concentration of Added ASPD (pM) | Theoretical Value (pM) | Measured Value (pM) | Recovery Rate % |
| H | 108 | 108 | 56.7 | 52.5 |
| M | 54 | 54 | 28.5 | 52.8 |
| L | 27.5 | 27.5 | 13.7 | 50.7 |
| N | 0 | 0 | 0 | 0 |

As can be seen from Table 2, ASPD could be measured when human plasma was used instead of human CSF. However, the recovery rate was reduced approximately by half. It is considered that this reduction was caused by the influence of plasma components.

Next, FIG. 7 shows the results obtained when human plasma was spiked with ASPD to prepare a sample, the sample was then diluted 2-fold, 4-fold, 8-fold, and 16-fold with a specimen diluent, and the samples with the dilution ratios of 1, 1/2, 1/4, 1/8, and 1/16 were subjected to measurement.

As can be seen from FIG. 7, dilution linearity was observed as a result of plotting the results obtained for the diluted samples. However, the inclination was reduced to 1/2 as compared with the inclination obtained when the buffer was used. It is considered that this was caused by the influence of plasma components.

The influence on the recovery rate in the human plasma samples could be eliminated by setting the sample dilution ratio to 1/16.

### 7. ASPD CLEIA [huASD2/mASD3]

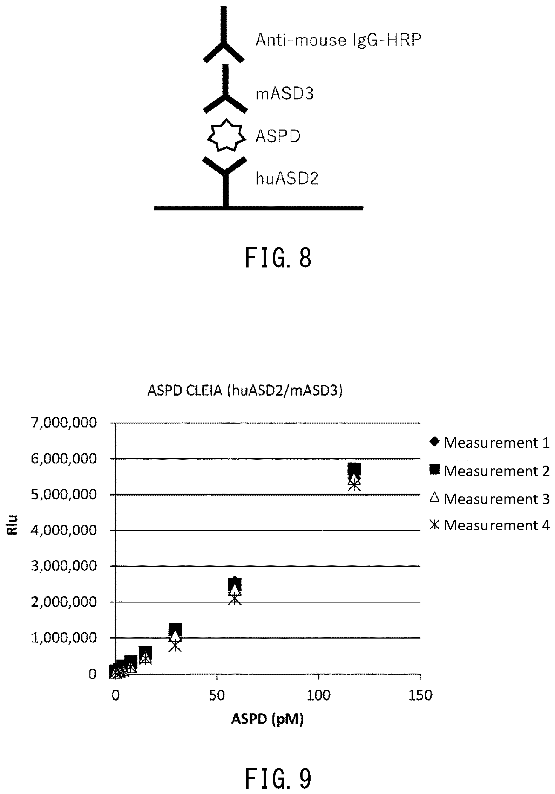

ASPD were measured in ASPD CLEIA in which humanized anti-ASPD monoclonal antibody huASD2 was used as an antibody immobilized on a solid phase and mouse anti-ASPD monoclonal antibody mASD3 and anti-mouse IgG-HRP were used (FIG. 8).

huASD2 is described in WO 2009/057664.

FIG. 9 shows an example of the results obtained. As can be seen from FIG. 9, as a result of four independent ASPD measurements, substantially the same luminescence values were obtained.

### 8. ASPD CLEIA [huASD2/biotinylated mASD3]

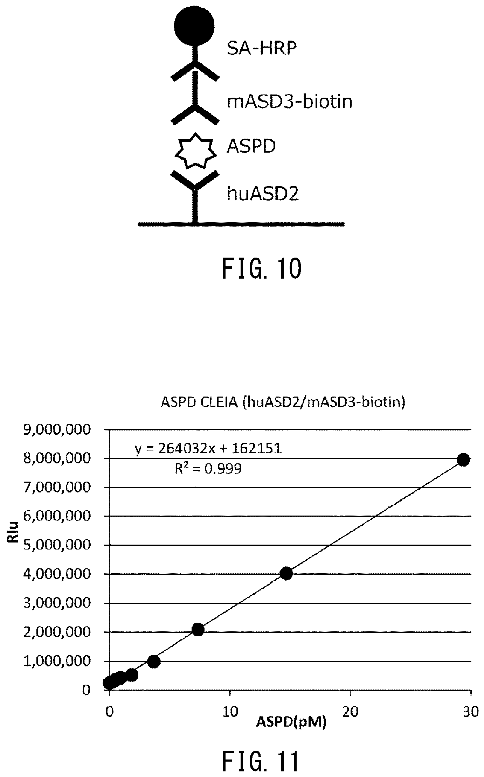

In order to improve the ASPD detection sensitivity, a system was constructed in which a biotinylated mASD3 antibody and HRP with streptavidin (SA) bound thereto were used (FIG. 10).

As a result of measuring ASPD in the huASD2/biotinylated mASD3 system, the detection sensitivity was improved as compared with the case where the measurement was performed using the huASD2/mASD3 system (FIG. 11).

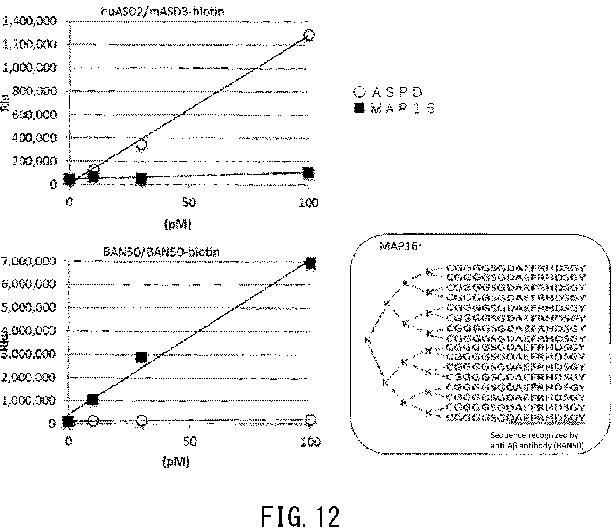

Moreover, the huASD2/biotinylated mASD3 system was highly specific for ASPD and hardly detected a multiple antigenic peptide having 16 molecules of Aβ1-10 bound thereto (MAP 16, FUJIFIIM Wako Pure Chemical Corporation) (FIG. 12). On the other hand, as a control, measurement was also performed using a BAN50/biotinylated BAN50 system (High Molecular Amyloid β Oligomer ELISA Kit, FUJIFILM Wako Pure Chemical Corporation). As a result, this system could hardly detected ASPD whereas it detected MAP 16 with high sensitivity (FIG. 12).

### 9. Antibody combinations capable of reducing plasma influence

Out of various combinations of a first antibody to be immobilized and a detection-side second antibody, combinations capable of reducing the influence of plasma were found.

ASPD CEILA systems were constructed with the following combinations of [first antibody/second antibody] and used to measure ASPD in plasma samples.

The combinations of [first antibody/second antibody] employed were [huASD2/biotinylated mASD3], [mASD3/biotinylated mASD3], [82E 1/biotinylated mASD3], [BAN50a/biotinylated mASD3], and [6E 10/biotinylated mASD3]. As samples, plasma samples containing ASPD and diluted at a dilution ratio of 1/4 were prepared and compared with a buffer sample.

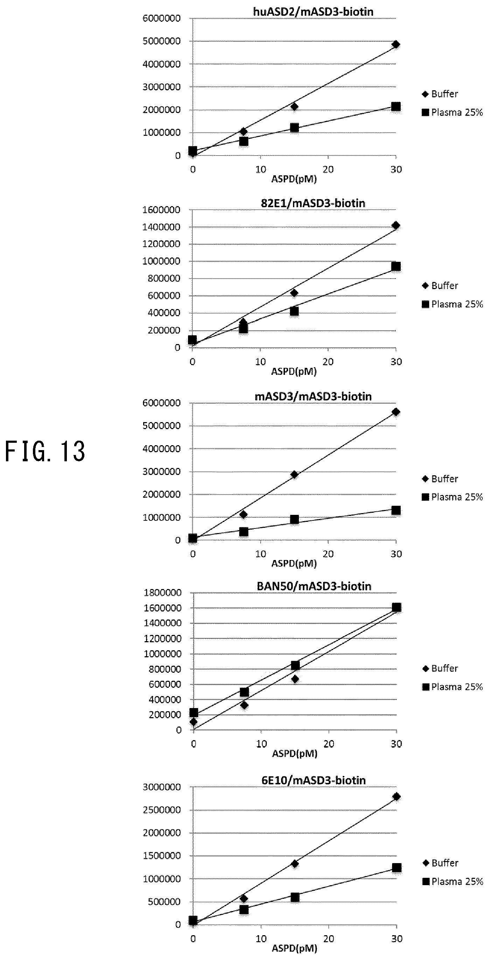

FIG. 13 shows an example of the results obtained.

As can be seen from FIG. 13, it was found that the influence of plasma components can be avoided most effectively when employing the combination of [BAN50a/biotinylated mASD3].

### 10. Measurement using blood samples from AD patients

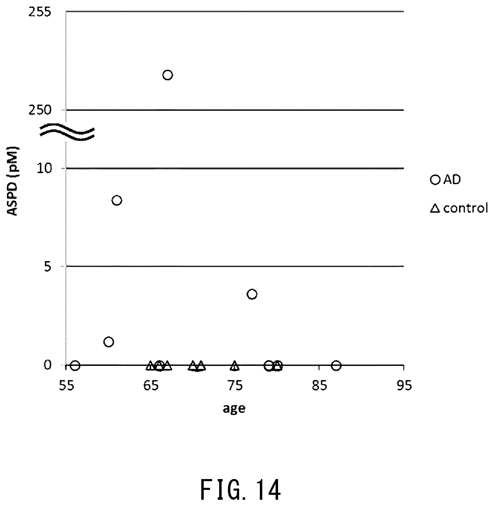

For ten plasma samples from patients diagnosed as having Alzheimer's disease (AD) (open circle) and ten plasma samples from healthy subjects (open triangle), measurement was performed in ASPD CLEIA employing the combination of [huASD2/biotinylated mASD3]. As a result, ASPD were detected from four samples from the AD patients. FIG. 14 shows the results obtained.

These results demonstrate that ASPD in blood can be measured using ASPD CLEIA.

## Claims

1. A method for analyzing amylospheroids (ASPD), comprising the steps of:
(1) bringing a biological sample that may contain ASPD into contact with an antibody (first antibody) that is capable of binding to ASPD and is bound to an insoluble carrier, thereby causing, when the biological sample contains ASPD, the ASPD to bind to the first antibody; and
(2) reacting an anti-ASPD monoclonal antibody (second antibody) with the ASPD that has bound to the first antibody in the step (1),
wherein the first antibody is a humanized anti-ASPD monoclonal antibody huASD2 or a monoclonal antibody BAN50a, and
the second antibody is a mouse monoclonal anti-ASPD antibody mASD3 or a labeled antibody that is mASD3 with a label.

2. The analysis method according to claim 1, further comprising the step of:
(3) reacting, with the second antibody that has bound to the ASPD in the step (2), an anti-mouse antibody having a reporter linked thereto, or reacting, with a label of the second antibody that has bound to the ASPD in the step (2), a binding partner of the label, having a reporter linked thereto.

3. The analysis method according to claim 1 or 2, wherein a substance that competes with amylospheroids (ASPD) against an ASPD-specific antibody is used as a reference material for ASPD, and the substance is a substance in which amyloid-β protein (AB) is cross-linked with a cross-linking agent,
wherein the cross-linking agent is a cross-linking agent that has a spacer arm length of between 7 Å and 50 Å inclusive or a cross-linking agent that has, as a spacer arm, not less than 1 and not more than 13 groups that are an oxyethylene group (-CH₂CH₂O-) and/or an oxypropylene group (-CH₂CH₂CH₂O-).

## Patentansprüche

1. Verfahren zum Analysieren von Amylospheroiden (ASPD), umfassend die Schritte:
(1) Inkontaktbringen einer biologischen Probe, die ASPD enthalten kann, mit einem Antikörper (erster Antikörper), der in der Lage ist, an ASPD zu binden und an einen unlöslichen Träger gebunden ist, wodurch, wenn die biologische Probe ASPD enthält, das ASPD an den ersten Antikörper gebunden wird; und
(2) Reagieren eines monoklonalen Anti-ASPD-Antikörpers (zweiter Antikörper) mit dem ASPD, das in Schritt (1) an den ersten Antikörper gebunden hat,
wobei der erste Antikörper ein humanisierter anti-ASPD monoklonaler Antikörper huASD2 oder ein monoklonaler Antikörper BAN50a ist, und
der zweite Antikörper ein monoklonaler Maus-Anti-ASPD-Antikörper mASD3 oder ein markierter Antikörper ist, der mASD3 mit einer Markierung ist.

2. Analyseverfahren nach Anspruch 1, weiter umfassend den Schritt:
(3) Reagieren eines Anti-Maus-Antikörpers, der einen daran gebundenen Reporter aufweist, mit dem zweiten Antikörper, der in Schritt (2) an das ASPD gebunden hat, oder Reagieren eines Bindungspartners des Markers, der einen daran gebundenen Reporter aufweist, mit einer Markierung des zweiten Antikörpers, der in Schritt (2) an das ASPD gebunden hat.

3. Analyseverfahren nach Anspruch 1 oder 2, wobei eine Substanz, die mit Amylospheroiden (ASPD) gegen einen ASPD-spezifischen Antikörper konkurriert, als Referenzmaterial für ASPD verwendet wird und die Substanz eine Substanz ist, in der das Amyloid-β-Protein (Aß) mit einem Vernetzungsmittel vernetzt ist,
wobei das Vernetzungsmittel ein Vernetzungsmittel ist, das eine Abstandhalterarmlänge zwischen 7 Å und 50 Å einschließlich aufweist oder ein Vernetzungsmittel, das als Abstandhalterarm nicht weniger als 1 und nicht mehr als 13 Gruppen aufweist, die eine Oxyethylengruppe (-CH₂CH₂O-) und/oder eine Oxypropylengruppe (-CH₂CH₂CH₂O-) sind.

## Revendications

1. Procédé pour analyser des amylosphéroïdes (ASPD), comprenant les étapes de :
(1) amenée d'un échantillon biologique qui peut contenir de l'ASPD en contact avec un anticorps (premier anticorps) qui est capable de se lier à l'ASPD et est lié à un support insoluble, provoquant ainsi, lorsque l'échantillon biologique contient de l'ASPD, la liaison de l'ASPD au premier anticorps ; et
(2) réaction d'un anticorps monoclonal anti-ASPD (deuxième anticorps) avec l'ASPD qui s'est lié au premier anticorps à l'étape (1),
dans lequel le premier anticorps est un anticorps monoclonal humanisé anti-ASPD huASD2 ou un anticorps monoclonal BAN50a, et
le deuxième anticorps est un anticorps monoclonal de souris anti-ASPD mASD3 ou un anticorps marqué qui est mASD3 avec un marqueur.

2. Procédé d'analyse selon la revendication 1, comprenant en outre l'étape de :
(3) réaction, avec le deuxième anticorps qui s'est lié à l'ASPD à l'étape (2), d'un anticorps anti-souris ayant un rapporteur lié à celui-ci, ou réaction, avec un marqueur du deuxième anticorps qui s'est lié à l'ASPD à l'étape (2), d'un partenaire de liaison du marqueur, ayant un rapporteur lié à celui-ci.

3. Procédé d'analyse selon la revendication 1 ou revendication 2, dans lequel une substance qui concurrence les amylosphéroïdes (ASPD) contre un anticorps spécifique à l'ASPD est utilisée comme matériau de référence pour l'ASPD, et la substance est une substance dans laquelle la protéine amyloïde-ß (Aß) est réticulée avec un agent de réticulation,
dans lequel l'agent de réticulation est un agent de réticulation qui a une longueur de bras d'espacement comprise entre 7 Å et 50 Å inclus ou un agent de réticulation qui a, en tant que bras d'espacement, pas moins de 1 et pas plus de 13 groupes qui sont un groupe oxyéthylène (-CH₂CH₂O-) et/ou un groupe oxypropylène (-CH₂CH₂CH₂O-).
